(19) Europäisches Patentamt
European Patent Office
Office européen des brevets

(11) EP 4 682 535 A1

(12) **EUROPEAN PATENT APPLICATION**
published in accordance with Art. 153(4) EPC

(43) Date of publication:
**21.01.2026 Bulletin 2026/04**

(21) Application number: **24770871.2**

(22) Date of filing: **12.03.2024**

(51) International Patent Classification (IPC):
***G01N 33/48*** *(2006.01)* ***C07K 16/18*** *(2006.01)*
***C12N 5/07*** *(2010.01)* ***C12N 15/115*** *(2010.01)*
***G01N 33/53*** *(2006.01)*

(52) Cooperative Patent Classification (CPC):
**C07K 16/18; C12N 5/06; C12N 15/115;**
**G01N 33/48; G01N 33/53**

(86) International application number:
**PCT/JP2024/009497**

(87) International publication number:
**WO 2024/190766 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.03.2023 JP 2023042210**

(71) Applicant: **NOF Corporation**
**Shibuya-ku**
**Tokyo 150-6019 (JP)**

(72) Inventors:
- **IMAMURA, Ryutaro**
  **Kawasaki-shi, Kanagawa 210-0821 (JP)**
- **YOSHIZAKI, Norihiro**
  **Kawasaki-shi, Kanagawa 210-0821 (JP)**
- **OHTAKE, Tomoyuki**
  **Kawasaki-shi, Kanagawa 210-0821 (JP)**

(74) Representative: **Weickmann & Weickmann PartmbB**
**Postfach 860 820**
**81635 München (DE)**

# (54) EXTRACELLULAR VESICLE DISSOCIATING AGENT AND EXTRACELLULAR VESICLE PURIFICATION METHOD

(57) The present invention provides an extracellular vesicle detachment agent containing a polymer containing a constitutional unit A represented by the following formula (1) and a constitutional unit B represented by the following formula (2) in a molar ratio of 0.8≤A/(A+B), having a number average molecular weight of 20,000 to 1,000,000, and further containing a separation carrier-binding site at an end thereof:

EP 4 682 535 A1

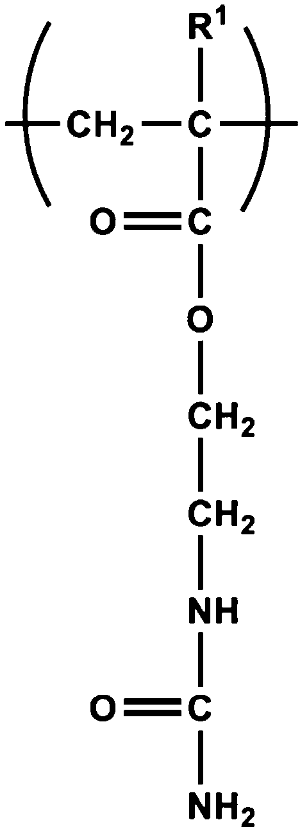

. . . (1)

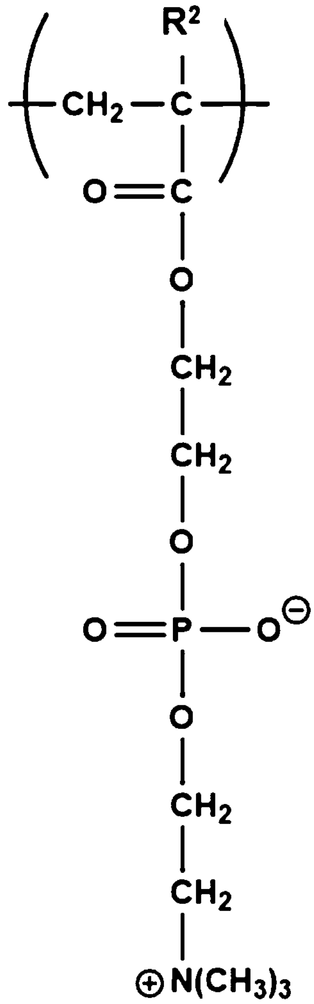

. . . (2)

(in the above-mentioned formula (1), $R^1$ is a hydrogen atom or a methyl group and in the above-mentioned formula (2), $R^2$ is a hydrogen atom or a methyl group).

## Description

[Technical Field]

The present invention relates to an extracellular vesicle detachment agent for separating an extracellular vesicle to be separated from a crude solution containing the extracellular vesicle to be separated, and the like.

[Background Art]

Extracellular vesicles are secreted by a variety of cells and are contained in blood (plasma, serum), urine, saliva, breast milk, and the like. They are also released into the culture supernatant of many cell lines. Known extracellular vesicles include exosomes with diameters of 30-150 nm and microvesicles with diameters of 30 to 1,000 nm, and they contain characteristic antigens and lipids such as phosphatidylserine (PS) on their membranes. Extracellular vesicles also contain proteins, nucleic acids such as messenger RNA (mRNA), microRNA (miRNA), low-molecular-weight double-stranded RNA (siRNA), and DNA, which reflect the characteristics of the cells from which they derive, and are believed to be involved in intercellular signaling when taken up by other cells.

In recent years, by utilizing the properties of extracellular vesicles that reflect the characteristics of the cells from which they derive, utilization thereof as disease biomarkers has been considered. In addition, studies are underway to use extracellular vesicles themselves as carriers in drug delivery systems, and to utilize useful proteins, that are contained in extracellular vesicles, for tissue regeneration. When using extracellular vesicles for these uses, it is important that the separated and recovered extracellular vesicles are intact and undamaged.

Furthermore, to achieve highly sensitive diagnostics and highly efficient drug delivery systems, it is necessary to recover specific extracellular vesicles with high purity.

As a method for separating and isolating extracellular vesicles from body fluids and the like, include ultracentrifugation method, density gradient centrifugation method, column method, immunoprecipitation method using magnetic particles, and the like are known. Of these, immunoprecipitation method using magnetic particles is a method that achieves high purity of the separated extracellular vesicles and high recovery rates. This method is also capable of isolating the above-mentioned specific extracellular vesicles.

As an immunoprecipitation method using magnetic particles, Non Patent Literature 1 describes a recovery method using Tim4 protein, which recognizes phosphatidylserine (PS) on extracellular vesicles. In this method, Tim4 protein bound to magnetic particles recognizes PS on extracellular vesicles and recovers the extracellular vesicles from a crude solution such as a body fluid. Thereafter, a chelating agent cleaves the bond between Tim4 protein and PS, thereby detaching the extracellular vesicles from the magnetic particles. While this method makes it possible to recover all extracellular vesicles in the crude solution, and the recovered extracellular vesicles are not damaged (intact), it has a problem in that isolation of a specific extracellular vesicle is not possible.

In addition, Patent Literature 1 describes a method for recovering extracellular vesicles by using a peptide with high affinity for phosphatidylserine (PS) on the extracellular vesicles. In this method, a peptide with affinity for PS and bound to magnetic particles recognizes the PS on extracellular vesicles and recovers the extracellular vesicles from the crude solution Thereafter, a 500 mM inorganic salt aqueous solution is used to cleave the peptide-PS bond, thereby detaching the extracellular vesicles from the magnetic particles. While this method makes it possible to recover all extracellular vesicles in the crude solution, and the recovered extracellular vesicles are not damaged (intact), it has a problem in that isolation of a specific extracellular vesicle is not possible.

As a method for separating extracellular vesicles containing specific antigens, a method using antibody-bound magnetic particles is generally known. While this method can separate extracellular vesicles containing specific antigens, it requires high-temperature treatment (about 70°C) or high-concentration ion treatment to cleave between antigen and antibody, which may problematically damage the extracellular vesicles.

[Citation List]

[Patent Literature]

[Patent Literature 1]
JP 2021-067576 A

[Non Patent Literature]

[Non Patent Literature 1]
Nakai, W. et al.: Sci. Rep., 6, 33935 (2016).

[Summary of Invention]

[Technical Problem]

As described above, no extracellular vesicle detachment agent is known that can recover specific extracellular vesicles from a crude solution such as body fluid without damaging them.

The problem of the present invention is to provide an extracellular vesicle detachment agent that can recover a specific extracellular vesicle without damage from a crude solution containing extracellular vesicles to be separated, and an extracellular vesicle purification method using the extracellular vesicle detachment agent.

[Solution to Problem]

The present inventors have conducted intensive studies in an attempt to achieve the aforementioned object and found that an extracellular vesicle detachment agent, in which the expansion and/or contraction of polymer chains due to temperature response of a temperature responsive polymer can be used as a driving force to detach extracellular vesicles to be detached from a separation carrier, that can bind to the separation carrier is useful. Furthermore, they have found that the aforementioned problem can be solved by optimizing the extracellular vesicle purification method using the above-mentioned extracellular vesicle detachment agent.

That is, the present invention provides at least the following [1] to [8].

[1] An extracellular vesicle detachment agent comprising a polymer comprising a constitutional unit A represented by the following formula (1) and a constitutional unit B represented by the following formula (2) in a molar ratio of $0.8 \leq A/(A+B)$, having a number average molecular weight of 20,000 to 1,000,000, and further comprising a separation carrier-binding site at an end thereof:

[Chem. 1]

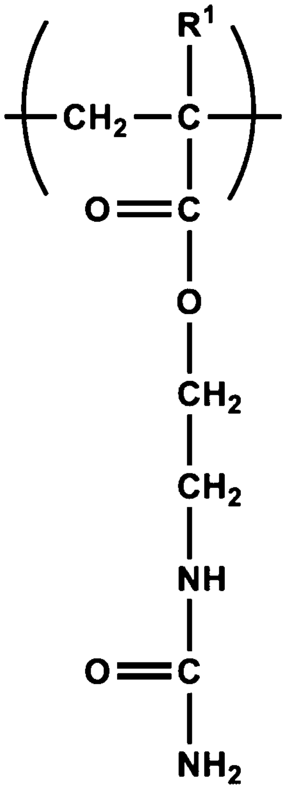 · · · (1)

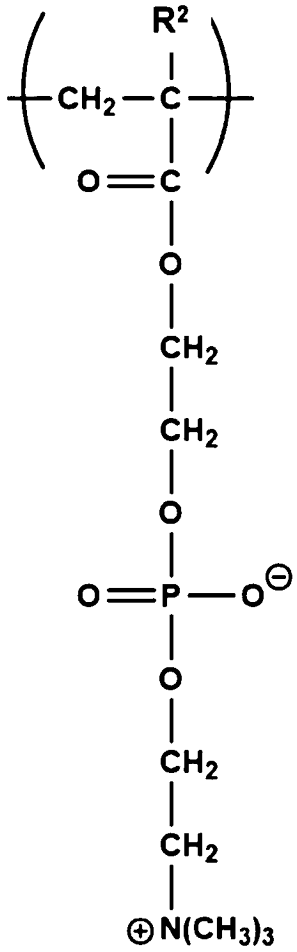 · · · (2)

(in the above-mentioned formula (1), $R^1$ is a hydrogen atom or a methyl group and in the above-mentioned formula (2), $R^2$ is a hydrogen atom or a methyl group).

[2] The extracellular vesicle detachment agent of [1], wherein the above-mentioned separation carrier-binding site is selected from a biotin residue, a streptavidin residue, an amino group, a carboxyl group, and a mercapto group.

[3] A method for purifying an extracellular vesicle, comprising the following [Step A] to [Step E] in this order:

[Step A] a step of binding a substance having a separation carrier-binding site and recognizing the extracellular vesicle to be separated to a separation carrier to obtain a separation carrier recognizing the extracellular vesicle to be separated

[Step B] a step of adding the separation carrier recognizing the extracellular vesicle to be separated to a crude solution containing the extracellular vesicles to be separated to allow for a reaction with the extracellular vesicles to be separated to obtain a complex of the extracellular vesicle to be separated-separation carrier

[Step C] a step of separating only the complex of the extracellular vesicle to be separated-separation carrier from the crude solution

[Step D] a step of adding the extracellular vesicle detachment agent of [1] or [2] to the complex of the extracellular vesicle to be separated-separation carrier, reacting them and then stirring the mixture to detach the extracellular vesicle to be separated from the complex of the extracellular vesicle to be separated-separation carrier

[Step E] a step of recovering the separation carrier and extracting the extracellular vesicle to be separated.

[4] The method for purifying an extracellular vesicle, of [3], wherein the above-mentioned substance recognizing the extracellular vesicle to be separated is an antibody, an antibody fragment, a peptide, an aptamer, or a charge-containing polymer.

[5] The method for purifying an extracellular vesicle, of [3], wherein the above-mentioned substance recognizing the extracellular vesicle to be separated is an antibody.

[6] The method for purifying an extracellular vesicle, of any of [3] to [5], wherein the above-mentioned separation carrier is a magnetic particle.

[7] The method for purifying an extracellular vesicle, of any of [3] to [6], wherein the above-mentioned stirring is pipetting.
[8] The method for purifying an extracellular vesicle, of any of [3] to [6], wherein the above-mentioned stirring includes 6 to 20 times of pipetting.

[Advantageous Effects of Invention]

The present invention can provide an extracellular vesicle detachment agent that can recover a specific extracellular vesicle without damage from a crude solution containing extracellular vesicles, an extracellular vesicle purification method using the agent, and the like.

[Description of Embodiments]

Preferred embodiments of the present invention are described in detail below.

<Extracellular vesicle detachment agent>

The above-mentioned extracellular vesicle detachment agent contains a polymer having a constitutional unit A represented by the following formula (1) and a constitutional unit B represented by the following formula (2), and the polymer has a separation carrier-binding site at an end thereof:

[Chem. 2]

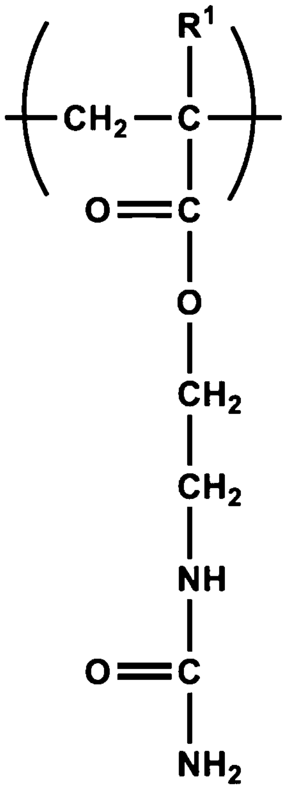

・・・(1)

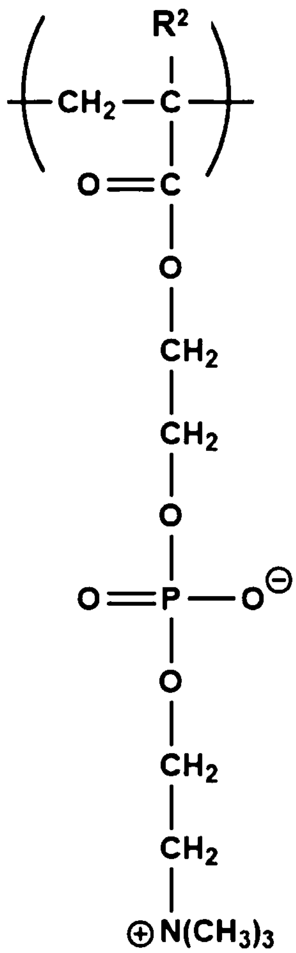

・・・(2)

(in the above-mentioned formula (1), $R^1$ is a hydrogen atom or a methyl group and in the above-mentioned formula (2), $R^2$ is a hydrogen atom or a methyl group).

The "constitutional unit" as used in the present specification refers to continuous repeats of the constitutional unit,

as well as intermittent repeats.

<Upper critical solution temperature (UCST) of extracellular vesicle detachment agent>

The above-mentioned upper critical solution temperature (UCST) is the boundary temperature between the temperature at which the above-mentioned polymer is insolubilized in water or a medium to form an insoluble phase, and the temperature at which the above-mentioned polymer dissolves in water or a medium to form a dissolved phase. The upper critical solution temperature (UCST) is not particularly limited but needs to be a temperature suitable for handling extracellular vesicles, which is preferably 2 to 60°C, more preferably 4 to 50°C, further preferably 4 to 40°C. At a temperature of 60°C or below, there is no risk of damage to extracellular vesicles. At a temperature of 4°C or above, there is no risk that the solution containing the extracellular vesicle detachment agent freezes, causing freeze damage to the extracellular vesicles.

The above-mentioned upper critical solution temperature (UCST) can be determined by dissolving the above-mentioned polymer in water or a medium at a concentration of at least 0.01 mg/mL, wherein the permeability when the polymer is completely dissolved is 100%, measuring the permeability of visible light at 500 nm in a quartz cell while decreasing the temperature, and determining the temperature at which the permeability starts to decrease when the temperature of the solution is decreased.

In the above-mentioned polymer, the molar ratio of the constitutional unit A represented by the above-mentioned formula (1) and the constitutional unit B represented by the above-mentioned formula (2) can be determined appropriately to exhibit the desired function. However, in order to set the upper critical solution temperature of the above-mentioned polymer within the range of 2 to 60°C, the molar ratio of the above-mentioned constitutional unit A and the above-mentioned constitutional unit B is preferably within the range of $0.3 \leq A/(A+B)$. More preferably the relationship of $0.4 \leq A/(A+B)$ is satisfied, more preferably the relationship of $0.5 \leq A/(A+B)$ is satisfied, further more preferably the relationship of $0.6 \leq A/(A+B)$ is satisfied, particularly preferably the relationship of $0.7 \leq A/(A+B)$ is satisfied, particularly more preferably the relationship of $0.8 \leq A/(A+B)$ is satisfied.

The above-mentioned polymer may be a copolymer with other monomers in addition to the monomer from which the constitutional unit A represented by the above-mentioned formula (1) above is derived and the monomer from which the constitutional unit B represented by the above-mentioned formula (2) above is derived. The upper critical solution temperature (UCST) can be adjusted according to the combination of the type of other monomers, molecular weight, and the like.

For example, the above-mentioned polymer may be a copolymer with other monomers listed below. To lower the upper critical solution temperature (UCST), for example, hydrophilic monomers can be selected as other monomers. Examples thereof include glycerin (meth)acrylate, (meth)acryloyloxyethylphosphate, N-methylcarboxybetaine (meth)acrylate, N-methylsulfobetaine (meth)acrylate, aminoethyl (meth)acrylate, N,N'-dimethylacrylamide, S-methylsulfoniumcarboxylic acid (meth)acrylate, ethylene glycol (meth)acrylate, and the like. In the use of the extracellular vesicle detachment agent, from the aspects of solubility in water and suppression of nonspecific adsorption, (meth)acryloyloxyethylphosphate, N-methylcarboxybetaine (meth)acrylate, N-methylsulfobetaine (meth)acrylate, aminoethyl (meth)acrylate, S-methylsulfoniumcarboxylic acid (meth)acrylate, and ethylene glycol (meth)acrylate are preferred.

The molecular weight of the above-mentioned polymer can be determined as appropriate by adjusting the polymerization conditions and the like so that the required properties can be exhibited, and it is generally about 1,000 to 5,000,000 in number average molecular weight. In order to increase the extracellular vesicle separation efficiency of the extracellular vesicle detachment agent of the present invention, it is preferably 10,000 to 2,000,000, more preferably 20,000 to 1,000,000. The molecular weight of the above-mentioned polymer is preferably not more than 5,000,000 because the solubility of the polymer in water increases to facilitate its use as an extracellular vesicle detachment agent.

The above-mentioned separation carrier-binding site is a site that can bind to a separation carrier and is not particularly limited as long as it is generally used in this field. Examples thereof include hydrophobic substituent, biotin residue, desthiobiotin residue, avidin residue, streptavidin residue, amino group, carboxyl group, mercapto group, glutathione residue, glutathione transferase residue, lectin residue, polysaccharide residues such as cyclodextrin residue and the like, adamantyl group, phenylboronic acid residue, and the like, preferably biotin residue, streptavidin residue, amino group, carboxyl group, mercapto group, and the like.

The content of the above-mentioned separation carrier-binding site is not particularly limited as long as the above-mentioned polymer is bound with the above-mentioned separation carrier. It is sufficient if 0.1 molecule to 2 molecules, preferably 0.2 molecule to 1.5 molecules, further preferably 0.5 molecule to 1 molecule, of the above-mentioned separation carrier-binding site is contained in 1 molecule of the above-mentioned polymer. When not more than 2 molecules are contained, crosslinking between separation carriers is less likely to occur, and 0.1 molecule or more is more preferred because the possibility of binding of the above-mentioned polymer to the above-mentioned separation carrier becomes higher.

As the above-mentioned polymer, for example, a polymer represented by the following formula (3) can be

mentioned:

[Chem. 3]

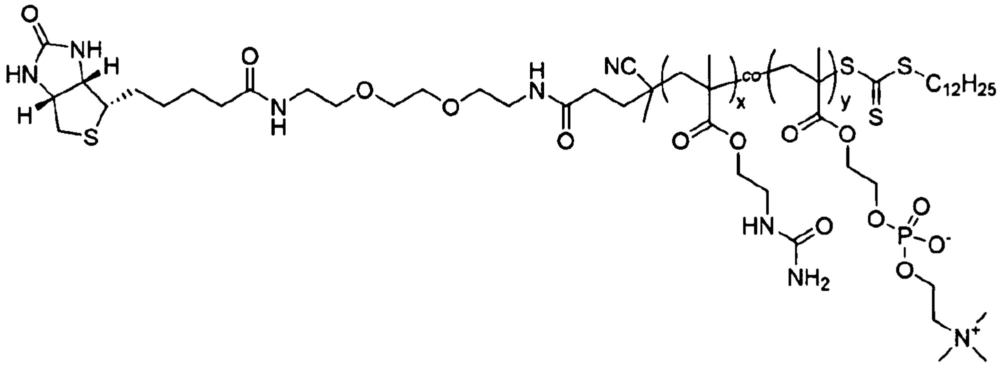

・・・(3)

wherein co indicates being a copolymer structure of two monomer units in the chemical formula, and x and y indicate composition ratios.

<Production method of polymer>

A part in the above-mentioned polymer except for the separation carrier-binding site can be produced, for example, by radical polymerization of 2-ureidoethyl methacrylate represented by the following formula (4) and 2-methacryloyloxyethylphosphoryl choline represented by the following formula (5).

[Chem. 4]

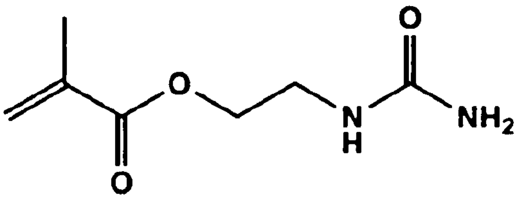

・・・(4)

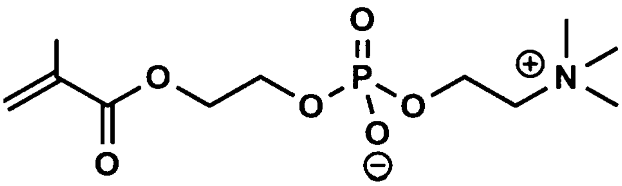

・・・(5)

Radical polymerization of the above-mentioned **2-**ureidoethyl methacrylate and the above-mentioned **2-**methacryloyloxyethylphosphoryl choline can also be copolymerization with other monomers.

Radical polymerization of the above-mentioned **2-**ureidoethyl methacrylate and the above-mentioned 2-methacryloyloxyethylphosphoryl choline (and the above-mentioned other monomers) can also be performed by bulk polymerization, or a solvent can also be added thereto, followed by performing the polymerization. The solvent is not particularly limited as long as the above-mentioned 2-ureidoethyl methacrylate and the above-mentioned 2-methacryloyloxyethylphosphoryl choline (and the above-mentioned other monomers) can be dissolved, and general solvents can be used. For example, the solvent is selected from polar aprotic solvents such as acetone, dioxane, N,N-dimethylformamide, dimethyl sulfoxide, tetrahydrofuran, and the like, polar protic solvents such as methanol, ethanol, water, and the like, and mixtures of these.

Radical polymerization of the above-mentioned 2-ureidoethyl methacrylate and the above-mentioned 2-methacryloyloxyethylphosphoryl choline (and the above-mentioned other monomers) can be performed by thermal polymerization or photopolymerization. The above-mentioned thermal polymerization can be performed, for example, using a thermal polymerization initiator. As the thermal polymerization initiator, for example, a peroxide radical initiator

(benzoyl peroxide, ammonium persulfate, etc.) or an azo radical initiator (azobisisobutyronitrile (AIBN), 2,2'-azobisdimethylvaleronitrile (ADVN), etc.), 2,2'-azobiscyanovaleric acid (ACVA), azobis[2-(2-imidazolin-2-yl)propane] dihydrochloride (VA-044), water-soluble or oil-soluble redox radical initiator (consisting of dimethylaniline and benzoyl peroxide), or the like can be used. The amount of the radical initiator to be used is preferably generally 0.01 to 10 parts by mass per total 100 parts by mass of the above-mentioned 2-ureidoethyl methacrylate and the above-mentioned 2-methacryloyloxyethylphosphoryl choline (and the above-mentioned other monomers). The polymerization temperature and polymerization time can be appropriately selected and determined according to the kind of the radical initiator, the presence or absence and the kind of other monomers, and the like. For example, when the radical polymerization of the above-mentioned 2-ureidoethyl methacrylate and the above-mentioned 2-methacryloyloxyethylphosphoryl choline (and the above-mentioned other monomers) is performed using ACVA, and the like, a polymerization temperature of 40 to 90°C and a polymerization time of about 2 to 48 hr are appropriate.

The above-mentioned photopolymerization can be performed, for example, by irradiation of ultraviolet (UV) at a wavelength of 254 nm or electron beam (EB) at an acceleration voltage of 150 to 300 kV, or the like. In this case, the use of photopolymerization initiator is optional, but is preferred from the aspect of reaction time. As the photopolymerization initiator, 2-hydroxy-2-methyl-1-phenyl-1-propanone, 1-hydroxycyclohexyl phenyl ketone, and the like can be mentioned. From the aspects of solubility and the like, 2-hydroxy-2-methyl-1-phenyl-1-propanone is preferred.

In the radical polymerization of the above-mentioned 2-ureidoethyl methacrylate and the above-mentioned 2-methacryloyloxyethylphosphoryl choline (and the above-mentioned other monomers), a chain transfer agent can also be used. As the above-mentioned chain transfer agent, 2-mercaptoethanol, 1-mercapto-2-propanol, 3-mercapto-1-propanol, p-mercaptophenol, mercaptoacetic acid, 2-mercaptopropionic acid, 3-mercaptopropionic acid, 2-mercaptonicotinic acid, and the like can be mentioned.

The radical polymerization of the above-mentioned 2-ureidoethyl methacrylate and the above-mentioned 2-methacryloyloxyethylphosphoryl choline (and the above-mentioned other monomers) can also be performed by a living radical polymerization method. Specifically, an atom transfer radical polymerization method (ATRP method), a reversible addition-fragmentation chain transfer polymerization method (RAFT polymerization method), a nitroxide-mediated polymerization method (NMP method), or the like can be used. In particular, for use of the raw materials of the extracellular vesicle detachment agent of the present invention, the reversible addition-fragmentation chain transfer polymerization method (RAFT polymerization method) is preferred because no metals are used and the enzyme activity is not reduced.

As the above-mentioned RAFT polymerization method, known methods can be utilized and, for example, the methods described in WO99/31144, WO98/01478, US Patent No. 6,153,705, and the like are effective. When the radical polymerization of the above-mentioned 2-ureidoethyl methacrylate and the above-mentioned 2-methacryloyloxyethylphosphoryl choline (and the above-mentioned other monomers) is performed using the RAFT polymerization method, it can be performed by adding a RAFT agent to general radical polymerization. The above-mentioned RAFT agent is selected from a biotin group-containing RAFT agent represented by the following formula (6), 4-cyanopentanoic acid dithiobenzoate, 2-cyano-2-propyl benzodithioate, benzyl benzodithioate, 2-phenyl-2-propyl benzodithioate, methyl 2-phenyl-2-(phenyl-carbonothioylthio)acetate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, 2-cyano-2-propyl dodecyl trithiocarbonate, 2-(dodecylthiocarbonylthioylthio)-2-methylpropionic acid, 4-cyano-4-(dodecylsulfanyl-thiocarbonyl)sulfanylpentanoic acid polyethylene glycol methyl ether ester, 2-(dodecylthiocarbonothioylthio)-2-methylpropionic acid-3-azido-1-propanol ester, benzyl 1H-pyrrole-1-carbodithioate, 2-cyanopropan-2-yl-N-methyl-N-pyridine-4-ylcarbodithioate, ethyl propionate-2-ethyl xanthate, and the like. From the aspect of the control of the polymerization of the above-mentioned 2-ureidoethyl methacrylate and the above-mentioned 2-methacryloyloxyethylphosphoryl choline (and the above-mentioned other monomers), a biotin group-containing RAFT agent represented by the following formula (6), 4-cyanopentanoic acid dithiobenzoate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, or 2-(dodecylthiocarbonothioylthio)-2-methylpropionic acid-3-azido-1-propanol ester is preferred. Furthermore, the above-mentioned 2-ureidoethyl methacrylate and the above-mentioned 2-methacryloyloxyethylphosphoryl choline can form random copolymers or block copolymers with the above-mentioned other monomers, but random copolymer is preferred to bring out the property of the above-mentioned extracellular vesicle detachment agent.

[Chem. 5]

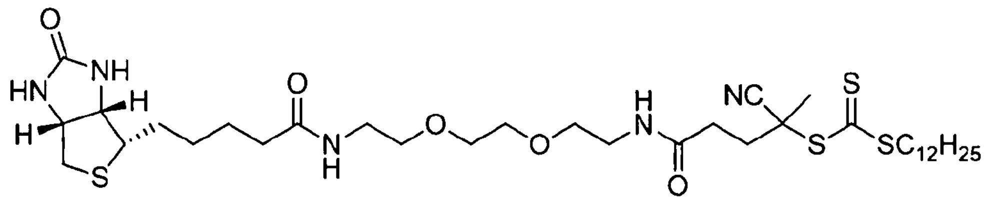

・・・(6)

<Method for including separation carrier-binding site at the end of polymer>

As a method for including the above-mentioned separation carrier-binding site at the end of a part in the above-mentioned polymer except for the separation carrier-binding site, addition, click reaction, amidation, esterification, thioesterification, carbonization, Schiff base formation reaction, reductive amination, radical-mediated coupling reaction, Diels Alder reaction, disulfidation, Michael addition reaction, ene-thiol reaction, aromatic boron compound-mediated coupling reaction, tertiary sulfonium formation reaction, quaternary ammonium formation reaction, and the like can be mentioned. Click reaction, amidation, or esterification is preferred due to their high versatility. The compound for constituting the above-mentioned separation carrier-binding site is not particularly limited, and examples thereof include a compound represented by the below-mentioned formula (6) and the like.

Furthermore, by using living radical polymerization method, the above-mentioned polymer having the above-mentioned separation carrier binding site at an end thereof can also be produced in a single step. Specifically, the atom transfer radical polymerization method (ATRP method), the reversible addition-fragmentation chain transfer polymerization method (RAFT polymerization method), the nitroxide-mediated polymerization method (NMP method), or the like can be used. In particular, for the use of the extracellular vesicle detachment agent of the present invention, the reversible addition-fragmentation chain transfer polymerization method (RAFT polymerization method) is preferred because no metals are used and the enzyme activity is not reduced.

As the above-mentioned RAFT polymerization method, known methods can be utilized and, for example, the methods described in WO99/31144, WO98/01478, US Patent No. 6,153,705, and the like are effective. When the above-mentioned separation carrier-binding site is introduced simultaneously with the radical polymerization of the above-mentioned 2-ureidoethyl methacrylate and the above-mentioned 2-methacryloyloxyethylphosphoryl choline (and the above-mentioned other monomers) using the RAFT polymerization method, it can be performed by adding a compound for constituting the separation carrier-binding site and a RAFT agent similar to those described above to general radical polymerization. In many cases, the RAFT agent also serves as the compound for constructing the separation carrier-binding site similar to that described above. The above-mentioned RAFT agent is selected from a biotin group-containing RAFT agent represented by the following formula (6), 4-cyanopentanoic acid dithiobenzoate, 2-cyano-2-propyl benzodithioate, benzyl benzodithioate, 2-phenyl-2-propyl benzodithioate, methyl 2-phenyl-2-(phenyl-carbonothioylthio)acetate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, 2-cyano-2-propyl dodecyl trithiocarbonate, 2-(dodecylthiocarbonylthioylthio)-2-methylpropionic acid, 4-cyano-4-(dodecylsulfanyl-thiocarbonyl)sulfanylpentanoic acid polyethylene glycol methyl ether ester, 2-(dodecylthiocarbonothioylthio)-2-methylpropionic acid-3-azido-1-propanol ester (N3-DTPA), benzyl 1H-pyrrole-1-carbodithioate, 2-cyanopropan-2-yl-N-methyl-N-pyridine-4-ylcarbodithioate, ethyl propionate-2-ethyl xanthate, and the like. Due to the easiness of introduction of a separation carrier-binding site into the end of the above-mentioned polymer, a biotin group-containing RAFT agent represented by the following formula (6), 4-cyanopentanoic acid dithiobenzoate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, or **2**-(dodecylthiocarbonothioylthio)-2-methylpropionic acid-3-azido-1-propanol ester is preferred.

[Chem. 6]

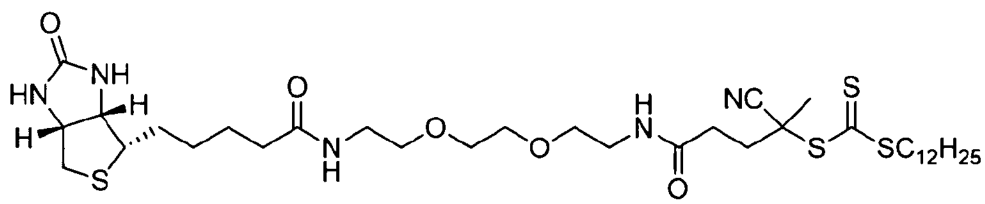

・・・(6)

<Extracellular vesicle detachment agent>

The extracellular vesicle detachment agent in the present invention is a material for detaching an extracellular vesicle to be separated from a separation carrier and contains at least the above-mentioned polymer. In addition, it generally contains water or medium in addition to the above-mentioned polymer, and may also contain additives such as chelating agent, protein, and the like for more efficient extracellular vesicle separation.

As the above-mentioned water, purified water, pure water, ion exchange water, and the like are preferred, and may be various buffers containing water. As various buffers, buffers normally used in this field can be used as long as they do not cause loss of physiological activity such as enzymatic activity and antigenicity of proteins. For example, phosphate buffer, tris buffer, Good's buffer, glycine buffer, borate buffer, and the like can be mentioned, and a mixture of these may also be used.

The above-mentioned medium is not particularly limited as long as it is suitable for the extracellular vesicle to be separated, and general cell culture medium can be used. Examples thereof include Dulbecco's modified Eagle MEM medium (DMEM), α-MEM medium, Roswell Park Memorial Institute (RPMI) medium, F12 medium, TC199 medium, GMEM medium, and the like. These may be mixed or supplements such as fetal bovine serum (FBS), glutamine, bovine serum albumin, human serum albumin, or even antibiotics may be added as necessary.

Examples of the above-mentioned additive include other reagents generally used in this field to improve extracellular vesicle separation efficiency, for example, chelating agents, saccharides, polysaccharides, proteins, salts, vitamins, surfactants, and the like.

Examples of the above-mentioned chelating agent include ethylenediaminetetraacetic acid (EDTA), 1,2-bis(O-aminophenoxide)ethane-N,N,N',N'-tetraacetic acid (BAPTA), bicine, trans-1,2-diaminocyclohexane-N,N,N',N'-tetraacetic acid (CyDTA), diethylenetriaminepentaacetic acid (DTPA), iminodiacetic acid, triethylenetetraminehexaacetic acid, and the like.

The above-mentioned saccharides refer to monosaccharides, disaccharides, oligosaccharides, and the like. Examples thereof include allose, glucose (grape sugar), altrose, mannose, galactose, psicose, fructose (fruit sugar), sorbose, ribose, xylose, arabinose, and the like as the monosaccharides, xylulose, trehalose, isotrehalose, maltose (malt sugar), cellobiose, isomaltose, and the like as the disaccharides, and fructooligosaccharide, galactooligosaccharide, lactosucrose, deoxyribose, fucose, rhamnose, glucuronic acid, galacturonic acid, glucosamine, galactosamine xylitol, sorbitol, ascorbic acid (vitamin C), glucuronolactone, gluconolactone, and the like as the oligosaccharides.

Examples of the above-mentioned polysaccharide include chitosan, dextran, cyclodextrin, hyaluronic acid, carboxymethylcellulose, hydroxypropylcellulose, hydroxyethylstarch, starch, pullulan, and the like.

Examples of the above-mentioned protein include bovine fetal serum (FBS), antifreeze peptide, bovine serum albumin, collagen, gelatin, casein, and the like.

Examples of the above-mentioned salt include amino acids such as glycine, alanine, serine, threonine, glutamic acid, aspartic acid, glutamine, asparagine, lysine, histidine, and the like, and amino acid salts, peptides such as glycyl glycine and the like, inorganic salts such as phosphate, borate, sulfate, Tris-salt, and the like, flavins, organic acids such as acetic acid, citric acid, malic acid, maleic acid, gluconic acid, and the like, and organic acid salts, and the like.

Examples of the above-mentioned vitamin include vitamin A, vitamin B1, vitamin B2, vitamin B3, vitamin B5, vitamin B6, vitamin B7, vitamin B9, vitamin B12, vitamin C, vitamin-like substances, and the like.

Examples of the above-mentioned surfactant include Triton, polyoxyethylene alkyl ether, Tween 20, Pluronic, and the like.

The content of the above-mentioned polymer contained in the above-mentioned extracellular vesicle detachment agent of the present invention is not particularly limited as long as it can be used for separation of extracellular vesicles. It is preferably not less than 0.01 mass %, more preferably not less than 0.05 mass %, of the extracellular vesicle detachment agent.

The above-mentioned additives contained in the extracellular vesicle detachment agent of the present invention are not particularly limited as long as they can be used for separation of extracellular vesicles. The content is preferably not less than 0.01 mass %, more preferably not less than 0.05 mass %, relative to the above-mentioned extracellular vesicle detachment agent.

<Separation carrier>

The material of the above-mentioned separation carrier is not particularly limited and plastic, polysaccharides, metal, metal with magnetism, ceramics, or the like is used. A plurality of these materials may be used. Examples of the plastic include acrylic polymers such as polymethyl methacrylate and the like, various silicone rubbers such as polydimethylsiloxane and the like, polystyrene, polypropylene, poly(ethylene terephthalate), polycarbonate, and the like.

The shape of the separation carrier may be, for example, plate, particle, and fiber shapes, as well as porous shape with holes, grooves, or protrusions formed in a plate-shaped or particle-shaped substrate. Among these, plate,

particle, fiber, and particle with magnetism (magnetic particle) shapes are preferred for ease of handling during extracellular vesicle separation, and magnetic particles are particularly preferred.

When the above-mentioned separation carrier is a magnetic particle, though free of particular limitation, the average particle size is preferably not less than 10 nm and less than 5000 nm, and in order to increase recognition of the object, an average particle size of not less than 20 nm and less than 3000 nm is particularly preferred. Examples of the material of the magnetic particles include magnetite, nickel oxide, ferrite, cobalt iron oxide, barium ferrite, carbon steel, tungsten steel, KS steel, rare earth cobalt magnet, hematite, and the like.

The surface of the above-mentioned separation carrier may have various functional groups (e.g., hydroxyl group, tosyl group, carboxyl group, amino group, epoxy group, mercapto group, sulfide group, polyhydric alcohol residue, and the like), and may also have on the surface residues of various compounds (e.g., residues of proteins such as streptavidin, gelatin, collagen, fibronectin, and the like, residues of polysaccharides such as dextran, pullulan, chitosan, cyclodextrin, and the like, residues of chelating agents such as ethylenediamine, and residues of surfactants such as polysorbate).

The combination of the above-mentioned separation carrier surface and the above-mentioned separation carrier-binding site is not particularly limited as long as they can be bound. The combination of a streptavidin residue and a biotin residue, the combination of a streptavidin residue and a desthiobiotin residue, the combination of a tosyl group and an amino group, the combination of an epoxy group and an amino group, the combination of a carboxyl group and an amino group, the combination of a cyclodextrin residue and an adamantyl group, the combination of a polyhydric alcohol residue and a phenylboronic acid residue, and the like can be mentioned. In view of easy availability, the combination of a streptavidin residue and a biotin residue, the combination of a streptavidin residue and a desthiobiotin residue, the combination of a tosyl group and an amino group, the combination of an epoxy group and an amino group, or the combination of a carboxyl group and an amino group is preferred, and the combination of a streptavidin residue and a biotin residue, the combination of a streptavidin residue and a desthiobiotin residue, or the combination of a tosyl group and an amino group is most preferred.

<Extracellular vesicle>

The above-mentioned extracellular vesicle to be separated is a particle covered with a membrane secreted by cells. Examples include exosomes with diameters of 30 to 150 nm, microvesicles with diameters of 30 to 1,000 nm, and apoptotic bodies with diameters of 500 to 2,000 nm.

As the above-mentioned cell, established cells for culture, fertilized egg and ovum cell of an animal (including human), and the like can be mentioned. In addition, animal (including human) cells such as stem cells (e.g., sperm cell, ES cell, iPS cell, mesenchymal stem cell, hematopoietic stem cell, neural stem cell, cord blood cell, and the like), hepatocyte, nerve cell, cardiomyocyte, vascular endothelial cell, vascular smooth muscle cell, blood cell, and the like or plant cells can also be mentioned. Furthermore, as the state of cells, suspension state and plate culture state, as well as the form of sheets, spheroids, organoids, and organs with higher-order structures can be mentioned.

<Extracellular vesicle purification method>

As a method for purifying extracellular vesicles by using a separation carrier, direct methods and indirect methods are known. The direct method involves binding a substance having a separation carrier-binding site and recognizing the extracellular vesicle to be separated to a separation carrier, and then reacting the same with the extracellular vesicles to be separated in a crude solution containing the extracellular vesicles to be separated (hereinafter to be also simply referred to as a "crude solution"). The indirect method involves reacting the extracellular vesicles to be separated in a crude solution with a substance having a separation carrier-binding site and recognizing the extracellular vesicle to be separated, and then reacting the same with a separation carrier. In the separation of extracellular vesicles, the direct method is more preferable from the aspect of separation efficiency.

Separation of extracellular vesicles to be separated from a crude solution containing the extracellular vesicles to be separated by using the extracellular vesicle detachment agent of the present invention can be performed by an extracellular vesicle purification method including the following [Step A] to [Step F] in this order.

[Step A] is a step in which a substance recognizing the extracellular vesicle to be separated is bound to the separation carrier to obtain a separation carrier recognizing the extracellular vesicle to be separated.

The above-mentioned extracellular vesicle to be separated refers to a population of extracellular vesicles obtained as a result of extracellular vesicle separation using the extracellular vesicle detachment agent of the present invention, and may be a single type or a population consisting of multiple types of extracellular vesicles.

The above-mentioned substance recognizing the extracellular vesicle to be separated is a substance that exhibits specific affinity for antigens or membrane constituents expressed inside or outside of extracellular vesicles, and any antibody, antibody derivative, fragmented antibody, or fragmented antibody derivative that is against antigen, peptides, cell adhesion receptor molecule, receptor for co-stimulatory molecule, charge-containing polymer, nucleic

acid, artificially engineered binding molecule, protein A, lectin, aptamer, and the like can be mentioned.

As the substance having the above-mentioned separation carrier-binding site and recognizing the extracellular vesicle to be separated, commercially available reagents can also be used. It can also be prepared separately according to the substance to be used which recognizes the extracellular vesicle to be separated. As methods for including the above-mentioned separation carrier-binding site in the above-mentioned substance recognizing the extracellular vesicle to be separated, addition, click reaction, amidation, esterification, thioesterification, carbonization, Schiff base formation reaction, reductive amination, radical-mediated coupling reaction, Diels-Alder reaction, disulfidation, Michael addition reaction, ene-thiol reaction, aromatic boron compound-mediated coupling reaction, tertiary sulfonium formation reaction, quaternary ammonium formation reaction, and the like can be mentioned. Click reaction, amidation, or esterification is preferred due to their high versatility.

The reaction between the above-mentioned substance having the separation carrier-binding site and recognizing the extracellular vesicle to be separated and the above-mentioned separation carrier is not particularly limited as long as it does not adversely affect the activity of the substance recognizing the extracellular vesicle to be separated. The reaction temperature is generally 2°C to 35°C, preferably 2°C to 30°C, more preferably 5°C to 25°C. Also, the reaction time is not particularly limited and it is generally 10 min to 2 hr, preferably 10 min to 1 hr, more preferably 10 min to 30 min.

By performing [Step A] above, a separation carrier recognizing the extracellular vesicle to be separated can be obtained. A specific example of the substance recognizing the extracellular vesicle to be separated and having a separation carrier-binding site in [Step A] is a biotinylated antibody, and a specific example of the separation carrier is a magnetic particle. That is, a specific example of [Step A] is a step in which a biotinylated antibody is reacted with streptavidin magnetic particles to obtain antibody-modified magnetic particles.

[Step B] is a step in which a separation carrier recognizing the extracellular vesicle to be separated is added to a crude solution containing the extracellular vesicles to be separated, and reacted with the extracellular vesicles to be separated to obtain a complex of the extracellular vesicle to be separated-separation carrier.

Examples of the above-mentioned crude solution include cell culture supernatant, blood, plasma, serum, saliva, urine, lacrimal fluid, sweat, breast milk, amniotic fluid, cerebrospinal fluid (spinal fluid), bone marrow fluid, pleural effusion, ascites, synovial fluid, aqueous humor, and vitreous fluid. From the aspect of ease of collection, cell culture supernatant, blood, plasma, serum, saliva, and urine are preferred.

The reaction between the above-mentioned separation carrier recognizing the extracellular vesicle to be separated and the above-mentioned extracellular vesicle to be separated is not particularly limited as long as it does not adversely affect the extracellular vesicle to be separated. The reaction temperature is generally 2°C to 35°C, preferably 2°C to 30°C, more preferably 5°C to 25°C. Also, the reaction time is not particularly limited and it is generally 10 min to 2 hr, preferably 10 min to 1 hr, more preferably 10 min to 30 min.

The reaction between the above-mentioned separation carrier recognizing the extracellular vesicle to be separated and the above-mentioned extracellular vesicle to be separated can be performed in, for example, an extracellular vesicle purification buffer. It is not particularly limited as long as it does not adversely affect the extracellular vesicle to be separated and buffers and media generally used in this field can be used. Examples thereof include phosphate buffer, tris buffer, Good's buffer, glycine buffer, borate buffer, and the like, and a mixture of these may also be used. Phosphate buffer is preferred. Also, Dulbecco's modified Eagle MEM medium (DMEM), $\alpha$-MEM medium, Roswell Park Memorial Institute (RPMI) medium, F12 medium, TC199 medium, GMEM medium, and the like can be mentioned. These may be mixed or supplements such as fetal bovine serum (FBS), glutamine, or antibiotics may also be added as necessary, but serum-free medium is preferred to avoid impairing the reactivity of the substance recognizing the the extracellular vesicle to be separated. In addition, various chelating agents can also be used to prevent extracellular vesicle aggregation. As the chelating agent, the same chelating agents as those recited as examples of the chelating agent as an additive in the above-mentioned extracellular vesicle detachment agent can be mentioned, with preference given to ethylenediaminetetraacetic acid (EDTA). The concentration of chelating agent in the extracellular vesicle purification buffer is 0.1 mM to 20 mM, preferably 0.5 mM to 10 mM, more preferably 1 mM to 5 mM. When the concentration of the chelating agent is not less than 0.1 mM, an extracellular vesicle aggregation suppressive effect is more easily expressed. On the other hand, when it is not more than 20 mM, damages on the extracellular vesicle are less likely to be exhibited.

Furthermore, in order to promote the reaction between the above-mentioned separation carrier recognizing the extracellular vesicle to be separated and the above-mentioned extracellular vesicle to be separated, polysaccharides, proteins, surfactants, and the like may also be added. As the above-mentioned polysaccharides, the same polysaccharides as those recited as examples of the polysaccharide as an additive in the above-mentioned extracellular vesicle detachment agent can be mentioned, and as the above-mentioned protein, the same proteins as those recited as examples of the protein as an additive in the above-mentioned extracellular vesicle detachment agent can be mentioned. As the above-mentioned surfactant, the same surfactants as those recited as examples of the surfactant as an additive in the above-mentioned extracellular vesicle detachment agent can be mentioned, and dextran, bovine serum albumin, or Tween20 is preferred, and bovine serum albumin is more preferred. Polysaccharides, proteins, surfactants, and the like in an extracellular vesicle purification buffer are not particularly limited as long as they can be used for extracellular vesicle

purification, but not less than 0.01 mass% is preferred, and not less than 0.05 mass% is more preferred. When polysaccharides, proteins, surfactants, and the like in an extracellular vesicle purification buffer are within the above-mentioned range, the above-mentioned additives are more likely to exert sufficient effects.

A complex of the extracellular vesicle to be separated-separation carrier can be obtained by performing the above [Step B]. A specific example of [Step B] is a reaction between antibody-modified magnetic particles and extracellular vesicles to be separated.

[Step C] is a step in which only a complex of the extracellular vesicle to be separated-separation carrier is separated from a crude solution.

A method of separating only the above-mentioned complex of the extracellular vesicle to be separated-separation carrier from a crude solution containing the extracellular vesicle to be separated varies depending on the separation carrier. For example, when the separation carrier is a magnetic particle, a method of collecting by magnetic force is preferred. When the separation carrier is a particle such as a latex particle, it can be precipitated by time change or by centrifugation. Furthermore, when the separation carrier is a flat plate, it can simply be removed from the above-mentioned extracellular vesicle purification buffer.

After the above-mentioned separation, it is preferable to perform washing and washing is not limited as long as it does not adversely affect the extracellular vesicle to be separated. For example, the above-mentioned extracellular vesicle purification buffer can also be used.

A complex of the extracellular vesicle to be separated-separation carrier can be separated by performing the above [Step C]. A specific example of [Step C] is a step of extracting the complex of the extracellular vesicle to be separated-separation carrier from a crude solution containing the extracellular vesicle to be separated by magnetic force, centrifugation, or the like.

[Step D] is a step in which the above-mentioned extracellular vesicle detachment agent is added to the complex of the extracellular vesicle to be separated-separation carrier, and the mixture is reacted and then stirred to detach the extracellular vesicles to be separated from the complex of the extracellular vesicle to be separated-separation carrier.

While the above-mentioned extracellular vesicle detachment agent can be directly added to the above-mentioned complex of the extracellular vesicle to be separated-separation carrier, the above-mentioned extracellular vesicle detachment agent may be mixed with the above-mentioned extracellular vesicle purification buffer and added as an extracellular vesicle detachment agent solution. Also, the reaction time is not particularly limited and it is generally 10 min to 2 hr, preferably 10 min to 1 hr, more preferably 10 min to 30 min.

The above-mentioned stirring method is not particularly limited as long as it does not adversely affect the extracellular vesicles. For example, vortexing, pipetting, inversion mixing, and stirring with a sample mixer are preferred, and pipetting is even more preferred from the aspect of being less likely to damage the extracellular vesicles.

The above-mentioned pipetting refers to the operation of aspirating and discharging a liquid containing the complex of the extracellular vesicle to be separated-separation carrier by using a measuring device such as Pipetman, syringe, or electric pipetter. The number of pipetting is not particularly limited as long as it does not adversely affect the extracellular vesicles. For example, a range of 6 to 50 times is preferred, and a range of 6 to 20 times is more preferred. When the number is 50 times or less, the risk of damage such as rupture or crushing of the extracellular vesicles becomes less, and when the number exceeds 5 times, extracellular vesicles are more easily detached from the separation carrier.

Furthermore, the temperature at which the above-mentioned extracellular vesicle detachment agent is added to the complex of the extracellular vesicle to be separated-separation carrier is preferably not more than the upper critical solution temperature (UCST) of the above-mentioned polymer, which is a constituent component of the extracellular vesicle detachment agent to be used, more preferably within the range of 2°C to the upper critical solution temperature (UCST) of the above-mentioned polymer, further preferably within the range of 5°C to the upper critical solution temperature (UCST) of the above-mentioned polymer because this range does not adversely affect extracellular vesicles. Before stirring, it is preferable to change the temperature of the solution obtained by the above-mentioned reaction to a temperature not less than the upper critical solution temperature (UCST) of the above-mentioned polymer. As used herein, "change the temperature" refers to heating from a temperature not more than the upper critical solution temperature (UCST) of the above-mentioned polymer, which is a constituent component of the extracellular vesicle detachment agent, to a temperature not less than the upper critical solution temperature (UCST). The temperature after heating is above the temperature at which the above-mentioned extracellular vesicle detachment agent was added in the above-mentioned reaction and not more than 60°C, preferably above the temperature at which the above-mentioned extracellular vesicle detachment agent was added in the above-mentioned reaction and not more than 50°C, more preferably above the temperature at which the above-mentioned extracellular vesicle detachment agent was added in the above-mentioned reaction and not more than 45°C. Heating at not more than 60°C reduces the risk of damage to the extracellular vesicles due to denaturation of proteins on the extracellular vesicle surface. In addition, when the temperature of the solution obtained in the above-mentioned reaction is raised to a temperature above the upper critical solution temperature (UCST) of the above-mentioned polymer, it is preferable to further react the obtained solution before stirring. In addition, the reaction time before stirring is not particularly limited and is generally 10 min to 2 hr, preferably 10 min to 1 hr, more

preferably 10 min to 30 min.

The extracellular vesicle to be separated can be separated from the complex of the extracellular vesicle to be separated-separation carrier by performing the above [Step D]. A specific example of [Step D] is a step of reacting a complex of the extracellular vesicle to be separated-separation carrier with an extracellular vesicle detachment agent, and stirring the mixture to stop the interaction between the separation carrier and the extracellular vesicle to be separated and detach the extracellular vesicle to be separated.

[Step E] is a step in which the separation carrier is recovered and the extracellular vesicle to be separated is extracted.

A method of recovering the above-mentioned separation carrier and extracting the extracellular vesicle to be separated varies depending on the separation carrier. For example, when the separation carrier is a magnetic particle, a method of collecting by magnetic force is generally used. When the separation carrier is a particle such as a latex particle, it can be precipitated by time change or by centrifugation. Furthermore, when the separation carrier is a flat plate, it can simply be removed from the above-mentioned extracellular vesicle purification buffer.

After the above-mentioned extraction, it is preferable to perform washing and washing is not limited as long as it does not adversely affect the extracellular vesicle to be separated. For example, the above-mentioned extracellular vesicle purification buffer can also be used.

The separation carrier can be recovered and the extracellular vesicle to be separated can be extracted by performing the above [Step E]. A specific example of [Step E] is a step of collecting the separation carrier by magnetic force, centrifugation or the like, recovering the separation carrier, and extracting the extracellular vesicle to be separated.

[Example]

The present invention is explained in detail in the following by referring to Examples. The present invention is not limited to the following Examples and the like.

(Synthetic Example 1: Synthesis of polymer A)

[Chem. 7]

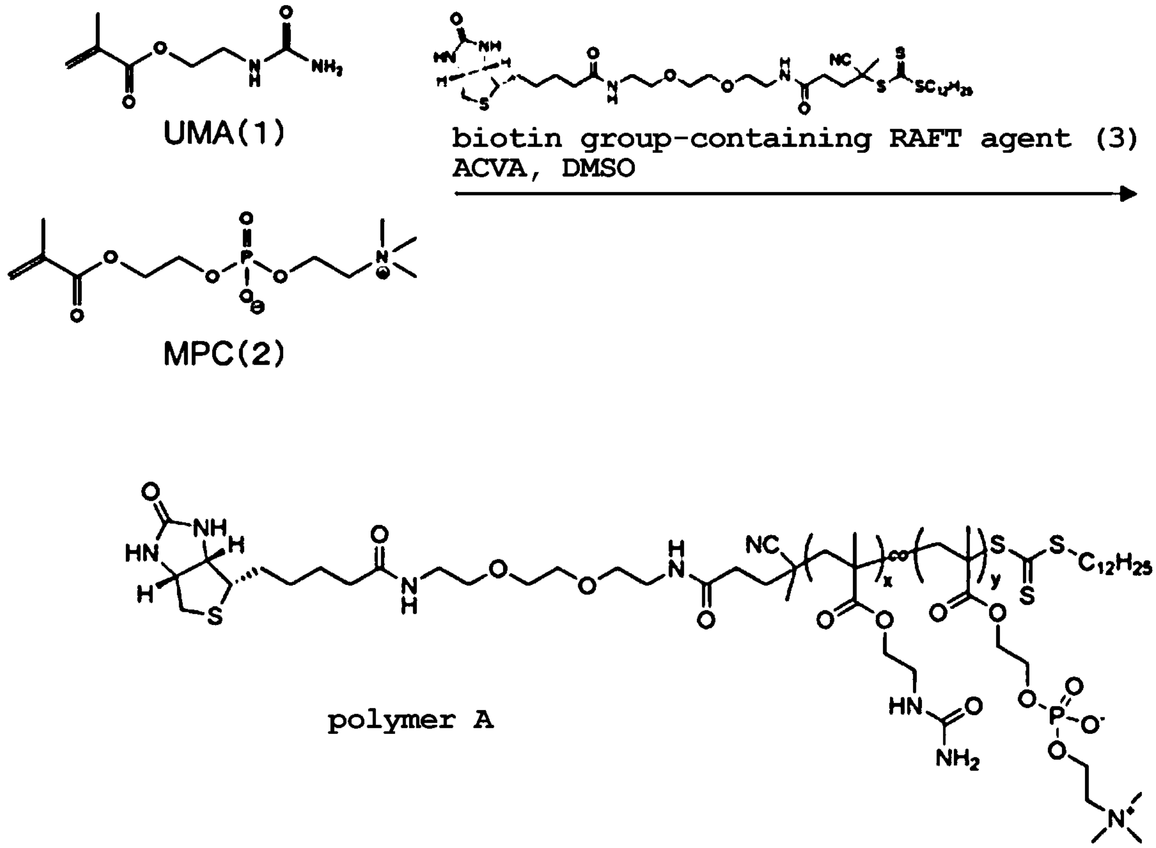


UMA(1)

MPC(2)

polymer A

wherein co indicates being a copolymer structure of two monomer units in the chemical formula, and x and y indicate composition ratios.

2-Ureidoethyl methacrylate (1) (UMA, 887 mg), 2-methacryloyloxyethylphosphoryl choline (2) (MPC, 97.1 mg), a biotin group-containing RAFT agent (3) (2.03 mg), and 4,4'-azo bis(4-cyanovaleric acid) (1.22 mg) were added into a test tube and dissolved in dimethyl sulfoxide (DMSO, 4.8 mL). After bubbling with a nitrogen gas, the reaction mixture was heated to 70°C and reacted for 6 hr. The polymerization solution was reprecipitated with acetone to give polymer A.

yield: 750 mg
composition ratio (molar ratio) x:y=94:6 (calculated from $^1$H NMR)

$^1$H-NMR($CD_3OD+D_2O$, 400 MHz)
4.1 ppm (Ureido: COO-$CH_2$) ,3.8-4.6 ppm(MPC:COO-$CH_2$, $CH_2$-$CH_2$,$H_2$), 3.6 ppm(MPC:$CH_2$-N), 3.4 ppm(Ureido:$CH_2$-$CH_2$), 3.2 ppm($N(CH_3)_3$),1.0-2.4 ppm(polymer main chain)

The number average molecular weight of the obtained polymer A was determined by gel permeation chromatography using a multi-angle light scattering detector (MALS) manufactured by WYATT as a detector. The pump used was LC-720AD manufactured by Shimadzu Corporation, the detector (differential refractometer) used was RID10A manufactured by Shimadzu Corporation, and the detector (UV) used was SPD-20A. Two columns of TSKGel G3000PWXL and TSKGel G5000PWXL (column size 4.6 mm×25cm) manufactured by Tosoh Corporation were connected and used. As the eluent, distilled water/acetonitrile (5/5 v/v) in which 100 mM sodium nitrate was dissolved was used. The measurement conditions were flow rate 0.6 ml/min, column temperature 40°C, sample concentration 2 mg/mL, injection volume 100 μL. As a result of gel permeation chromatography measurement, the number average molecular weight of the polymer A was 309,000.

The upper critical solution temperature (UCST) of the obtained polymer A was measured by an ultraviolet visible spectrophotometer. The polymer A was dissolved in a extracellular vesicle purification buffer (phosphate-buffered saline

(PBS)+0.1% bovine serum albumin (BSA)+2 mM EDTA) at a concentration of 2.5 mg/mL. The solution was placed in a quartz cell, the solution temperature was changed within the range of 70°C to 5°C, and the 500 nm visible light permeability (%) of the solution was measured by the ultraviolet visible spectrophotometer. The permeability (%) of the solution was calculated from the following formula. As a result, the upper critical solution temperature (UCST) of the polymer A was 20°C.

[Math. 1]

$$\text{transmittance (\%)} = 10^{(-\text{absorbance})}$$

(Preparation Example 1: Preparation of extracellular vesicle detachment agent solution A)

Polymer A was dissolved in an extracellular vesicle purification buffer (phosphate-buffered saline (PBS)+0.1% bovine serum albumin (BSA)+2 mM EDTA) at a concentration of 0.8 mg/mL to prepare extracellular vesicle detachment agent solution A.

<Example 1-1>

(Preparation of separation carrier recognizing extracellular vesicle to be separated)

Dynabeads (registered trademark) M-280 Streptavidin (0.5 mg) manufactured by Thermo Fisher as a separation carrier and biotinylated CD9 antibody (5 μg) manufactured by GeneTex as a substance recognizing the extracellular vesicle to be separated were mixed and reacted at room temperature for 20 min to obtain CD9 antibody-carrying magnetic particles as a separation carrier recognizing the extracellular vesicle to be separated.

(Pre-treatment of human serum)

Human serum manufactured by Merck was centrifuged at 4°C, 10,000×g for 10 min.. The human serum supernatant was diluted 5-fold with PBS(-) to prepare a human serum solution.

(Extracellular vesicle purification test from human serum solution)

Human serum solution (200 μL) was dispensed into an Eppendorf tube, CD9 antibody-carrying magnetic particles (0.2 mg) were added thereto, and the mixture was shaken at room temperature for 20 min. The magnetic particles were collected by magnetic force, the supernatant was removed, 200 μL of an extracellular vesicle purification buffer (phosphate-buffered saline (PBS)+0.1% bovine serum albumin (BSA)+2 mM EDTA) was added thereto, and the particles were washed. After washing three times, the particles were resuspended in 200 μL of the extracellular vesicle purification buffer, and cell detachment solution A was added thereto at 4°C. The mixture was reacted at 4°C for 15 min, and then reacted at 37°C for 15 min. After the reaction, the mixture was pipetted 20 times, collecting by magnetic force was performed, and the supernatant was recovered (measurement sample).

(Analysis of extracellular vesicle)

The numbers of extracellular vesicles in the human serum solution and in the measurement sample were analyzed using NanoSight LM10 manufactured by Malvern. As for the number of extracellular vesicles, the number of particles within the range of particle size 50 to 150 nm was counted, and it was calculated relative to the number of extracellular vesicles in the human serum solution before separation operation as 1. The separated extracellular vesicles were fixed with glutaraldehyde and then observed under an electron microscope using a transmission microscope manufactured by JEOL. Evaluation using the electron microscope was performed by observing the shape of the extracellular vesicles to analyze whether they were nearly spherical and not damaged. The analysis results of the extracellular vesicles are shown in Table 1.

<Example 1-2>

In the same manner as in Example 1-1 except that pipetting was performed five times in the extracellular vesicle purification test from human serum solution, an extracellular vesicle purification test was performed. The analysis results of the extracellular vesicles are shown in Table 1.

<Example 1-3>

In the same manner as in Example 1-1 except that pipetting was performed 50 times in the extracellular vesicle purification test from human serum solution, an extracellular vesicle purification test was performed. The analysis results of the extracellular vesicles are shown in Table 1.

<Comparative Example 1-1>

In the same manner as in Example 1-1 except that an extracellular vesicle detachment agent was not used, an extracellular vesicle purification test was performed. The analysis results of the extracellular vesicles are shown in Table 1.

<Comparative Example 1-2>

In the same manner as in Example 1-1 except that pipetting was not performed in the extracellular vesicle purification test from a human serum solution, an extracellular vesicle purification test was performed. The analysis results of the extracellular vesicles are shown in Table 1.

<Example 1-4>

(Pre-treatment of human serum)

In the same manner as in Example 1-1, human serum was pretreated.

(Extracellular vesicle purification test from human serum solution)

Human serum solution (200 μL) was dispensed into an Eppendorf tube, biotinylated CD9 antibody (2 μg) was added thereto, and the mixture was shaken at room temperature for 60 min. Dynabeads (registered trademark) M-280 Streptavidin (0.2 mg) was added thereto, and the mixture was shaken at room temperature for 60 min. The magnetic particles were collected by magnetic force, the supernatant was removed, 200 μL of an extracellular vesicle purification buffer (phosphate-buffered saline (PBS)+0.1% bovine serum albumin (BSA)+2 mM EDTA) was added thereto, and the particles were washed. After washing three times, the particles were resuspended in 200 μL of the extracellular vesicle purification buffer, and cell detachment solution A was added thereto at 4°C. The mixture was reacted at 4°C for 15 min, and then reacted at 37°C for 15 min. After the reaction, the mixture was pipetted 20 times, collecting by magnetic force was performed, and the supernatant was recovered (measurement sample).

The analysis of the amount of extracellular vesicles was performed as in Example 1-1. The analysis results of the extracellular vesicles are shown in Table 1.

From the above results, it was suggested that extracellular vesicles can be highly efficiently obtained by separating the extracellular vesicles by using the extracellular vesicle detachment agent of the present invention. In addition, the direct method was more preferable in terms of recovery rate improvement.

Furthermore, electron microscopic observation of extracellular vesicles obtained using the extracellular vesicle detachment agent of the present invention suggested that the obtained extracellular vesicles were not damaged.

The present inventors have conducted intensive studies in an attempt to achieve the aforementioned object and found that an extracellular vesicle detachment agent, in which the expansion and/or contraction of polymer chains due to temperature response of a temperature responsive polymer can be used as a driving force to detach extracellular vesicles to be detached from a separation carrier, that can bind to the separation carrier is useful. Furthermore, they have found that the aforementioned problem can be solved by optimizing the extracellular vesicle purification method using the above-mentioned extracellular vesicle detachment agent.

[Table 1]

| | extracellular vesicle detachment agent | stirring | order of steps | relative extracellular vesicle number | evaluation by electron microscope |
|---|---|---|---|---|---|
| human serum solution | - | | | 1 | - |
| Example 1-1 | extracellular vesicle detachment agent A | stirred by pipetting 20 times | direct method | 0.41 | ○ |

(continued)

| | extracellular vesicle detachment agent | stirring | order of steps | relative extracellular vesicle number | evaluation by electron microscope |
|---|---|---|---|---|---|
| Example 1-2 | extracellular vesicle detachment agent A | stirred by pipetting 5 times | direct method | 0.12 | ○ |
| Example 1-3 | extracellular vesicle detachment agent A | stirred by pipetting 50 times | direct method | 0.38 | Δ |
| Comparative Example 1-1 | - | stirred by pipetting 20 times | direct method | 0.02 | ○ |
| Comparative Example 1-2 | extracellular vesicle detachment agent A | not stirred | direct method | 0.05 | ○ |
| Example 1-4 | extracellular vesicle detachment agent A | stirred | indirect method | 0.23 | ○ |
| ○: nearly spherical and not damaged<br>Δ: partially crushed and aggregated | | | | | |

[Industrial Applicability]

The present invention provides an extracellular vesicle detachment agent that can recover a specific extracellular vesicle without damage from a crude solution containing extracellular vesicles, and an extracellular vesicle purification method using the agent.

This application is based on a patent application No. 2023-042210 filed in Japan (filing date: March 16, 2023), the contents of which are incorporated in full herein.

**Claims**

1. An extracellular vesicle detachment agent comprising a polymer comprising a constitutional unit A represented by the following formula (1) and a constitutional unit B represented by the following formula (2) in a molar ratio of $0.8 \leq A/(A+B)$, having a number average molecular weight of 20,000 to 1,000,000, and further comprising a separation carrier-binding site at an end thereof:

[Chem. 1]

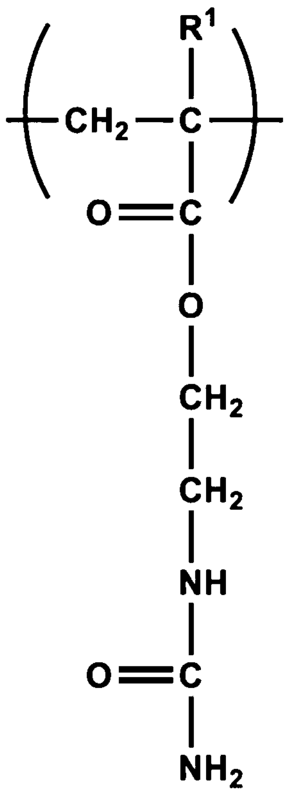

· · · (1)

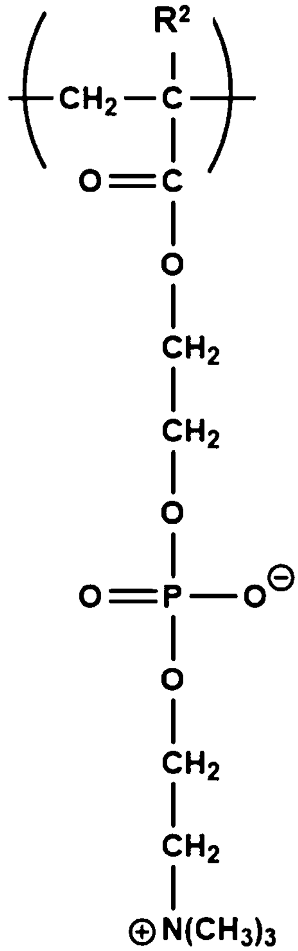

· · · (2)

(in the formula (1), $R^1$ is a hydrogen atom or a methyl group and in the formula (2), $R^2$ is a hydrogen atom or a methyl group).

2. The extracellular vesicle detachment agent according to claim 1, wherein the separation carrier-binding site is selected from a biotin residue, a streptavidin residue, an amino group, a carboxyl group, and a mercapto group.

3. A method for purifying an extracellular vesicle, comprising the following [Step A] to [Step E] in this order:

[Step A] a step of binding a substance having a separation carrier-binding site and recognizing the extracellular vesicle to be separated to a separation carrier to obtain a separation carrier recognizing the extracellular vesicle to be separated
[Step B] a step of adding the separation carrier recognizing the extracellular vesicle to be separated to a crude solution containing the extracellular vesicles to be separated to allow for a reaction with the extracellular vesicles to be separated to obtain a complex of the extracellular vesicle to be separated-separation carrier
[Step C] a step of separating only the complex of the extracellular vesicle to be separated-separation carrier from the crude solution
[Step D] a step of adding the extracellular vesicle detachment agent according to claim 1 or 2 to the complex of the extracellular vesicle to be separated-separation carrier, reacting them and then stirring the mixture to detach the extracellular vesicle to be separated from the complex of the extracellular vesicle to be separated-separation carrier
[Step E] a step of recovering the separation carrier and extracting the extracellular vesicle to be separated.

4. The method for purifying an extracellular vesicle, according to claim 3, wherein the substance recognizing the extracellular vesicle to be separated is an antibody, an antibody fragment, a peptide, an aptamer, or a charge-containing polymer.

5. The method for purifying an extracellular vesicle, according to claim 3, wherein the substance recognizing the extracellular vesicle to be separated is an antibody.

6. The method for purifying an extracellular vesicle, according to any one of claims 3 to 5, wherein the separation carrier is a magnetic particle.

7. The method for purifying an extracellular vesicle, according to any one of claims 3 to 5, wherein the stirring is pipetting.

8. The method for purifying an extracellular vesicle, according to any one of claims 3 to 5, wherein the stirring includes 6 to 20 times of pipetting.

## INTERNATIONAL SEARCH REPORT

International application No.
**PCT/JP2024/009497**

### A. CLASSIFICATION OF SUBJECT MATTER

***G01N 33/48***(2006.01)i; ***C07K 16/18***(2006.01)i; ***C12N 5/07***(2010.01)i; ***C12N 15/115***(2010.01)i; ***G01N 33/53***(2006.01)i
FI: G01N33/48 A; G01N33/53 U; C12N5/07; C07K16/18; C12N15/115 Z

According to International Patent Classification (IPC) or to both national classification and IPC

### B. FIELDS SEARCHED

Minimum documentation searched (classification system followed by classification symbols)

G01N33/48; C07K16/18; C12N5/07; C12N15/115; G01N33/53

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2023/276937 A1 (NOF CORPORATON) 05 January 2023 (2023-01-05) claims, paragraphs [0062], [0135]-[0157], [0159]-[0173] | 1-8 |
| Y | JP 2021-067576 A (HIROSHIMA UNIVERSITY) 30 April 2021 (2021-04-30) claims, paragraphs [0016], [0048], fig. 1 | 1-8 |
| Y | WO 2015/068772 A1 (JSR CORPORATION) 14 May 2015 (2015-05-14) claims, paragraph [0056] | 1-8 |
| A | WO 2011/118587 A1 (KYUSHU UNIVERSITY) 29 September 2011 (2011-09-29) entire text, all drawings | 1-8 |
| A | JP 2023-6594 A (NOF CORPORATON) 18 January 2023 (2023-01-18) entire text, all drawings | 1-8 |
| A | JP 2015-174962 A (TOKYO INSTITUTE OF TECHNOLOGY) 05 October 2015 (2015-10-05) entire text, all drawings | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 May 2024** | **28 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/009497**

**C. DOCUMENTS CONSIDERED TO BE RELEVANT**

| Category* | Citation of document, with indication, where appropriate, of the relevant passages | Relevant to claim No. |
|---|---|---|
| A | FUJIHARA, A. et al., Preparation of upper critical solution temperature (UCST) responsive diblock copolymers bearing pendant ureido groups and their micelle formation behavior in water. Soft Matter, 2015, vol. 11, pp. 5204-5213, DOI: 10.1039/c5sm00499c entire text, all drawings | 1-8 |

Form PCT/ISA/210 (second sheet) (July 2022)

**INTERNATIONAL SEARCH REPORT**
**Information on patent family members**

International application No.
**PCT/JP2024/009497**

| Patent document cited in search report | Publication date (day/month/year) | Patent family member(s) | Publication date (day/month/year) |
|---|---|---|---|
| WO 2023/276937 A1 | 05 January 2023 | (Family: none) | |
| JP 2021-067576 A | 30 April 2021 | (Family: none) | |
| WO 2015/068772 A1 | 14 May 2015 | US 2016/0349246 A1<br>claims, paragraphs [0094]-[0095]<br>CN 105723221 A | |
| WO 2011/118587 A1 | 29 September 2011 | US 2013/0012597 A1<br>EP 2551285 A1 | |
| JP 2023-6594 A | 18 January 2023 | (Family: none) | |
| JP 2015-174962 A | 05 October 2015 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021067576 A
- WO 9931144 A
- WO 9801478 A
- US 6153705 A
- JP 2023042210 A

### Non-patent literature cited in the description

- **NAKAI, W. et al.** *Sci. Rep.*, 2016, vol. 6, 33935